# EUROPEAN PATENT APPLICATION

(11) **EP 4 674 476 A1**
(43) Date of publication of application: **07.01.2026**
(21) Application number: 24186024.6
(22) Date of filing: 02.07.2024
(51) Int. Cl.: A61N 2/00

(54) **MAGNETIC FIELD VARIATION THERAPY FOR ENHANCING THERAPEUTIC AND DISINFECTION APPLICATIONS**

(71) Applicant: Azyro SA, 2339 Luxembourg (LU)
(72) Inventor: SERVAES, Jan, 2339 Luxembourg (LU); LASKARATOU, Danai, 2339 Luxembourg (LU); BENKHAI, Hicham, 2339 Luxembourg (LU); KRASS, Volker, 2339 Luxembourg (LU)
(74) Representative: Grund, Martin

(57) **Abstract**

The present disclosure refers to an antiseptic or antibiotic agent for use in treating a wound in a subject, wherein the subject is to receive a magnetic field treatment at the wound before, after or concurrently with the application of the agent, and wherein the magnetic field treatment comprises at least two phases of periodic waves, wherein in each consecutive phase a different type of periodic wave is applied, wherein the periodic waves differ in their waveform, their frequency and/or their intensity.

## Description

### TECHNICAL FIELD OF THE INVENTION

The disclosed invention lies in the field of magnetic fields used in antimicrobial therapy and disinfection/sterilization. More precise, the invention relates to treatment options to disinfect a wound or tissue by selectively eradicating bacteria based on the application of a magnetic field variation treatment to the infected wound in combination with the application of an antimicrobial agent. The technology encompasses the application of magnetic fields with changing parameters to enhance the efficacy of antibacterial agents, allowing for the treatment of resistant or multi-resistant bacterial strains.

### BACKGROUND OF THE INVENTION

Infective complications are a major factor contributing to wound chronicity and can be associated with significant morbidity and mortality, becoming an increasing burden for patients and healthcare systems. Such disease states lead to significant deterioration of life or, if not treated appropriately, to death. The infective complication of wound ulcerations impedes or stops the process of wound healing and is responsible for persistence or chronicity of a wound. In the past, such infections were treated locally by means of antibiotic-containing creams or ointments. However, local application of antibiotics has contributed to the rise of antibiotic-resistant pathogens. Due to this and other disadvantages related to the use of locally administered antibiotics, they are no longer indicated for chronic wound care. In cases of infected wounds, systemic antibiotic therapy is now recommended and serves to prevent bacterial penetration from the wound bed to deeper tissues including hematogenous spread. Thus, in local wound treatment, options include debridement of surgical, biological or chemical nature, intravenous antibiotic therapy, and antiseptic application. However, methicilin-resistant strains of *Staphylococcus aureus* (referred to as MRSA) have developed resistance to most β-lactam antibiotics including penicilins, cephalosporins, and carbapenems, as well as other antibiotic classes. This substantial level of resistance has made MRSA a major concern in healthcare.

The application of antiseptics instead of antibiotics has become progressively common in skin and wound therapy, especially in biofilm-related infections. Antiseptics are locally administered antimicrobials, and due to non-specific mechanisms of action, the use of a majority of them does not lead to microbial resistance. There are two major mechanisms of action for antiseptics. The first involves charged molecules in the antiseptic binding to bacterial cell walls and membranes, resulting in destruction of these structures. This is followed by leakage of cytoplasmic components into the environment, enzymatic malfunctions, and to bacterial cell death. One of the most potent antiseptics having such effect is octenidine dihydrochloride (Octenidine, OCT), other major antiseptics are chlorhexidine (CHX) and polyhexanide (PHMB). The second mechanism for antiseptic activity relies on the ability to destroy and denature bacterial proteins. The most common antiseptic displaying this mechanism of action is povidone-iodine (PVP-I). Other mechanisms include binding and disrupting of microbial DNA, as seen with the commonly used antiseptic ethacridine lactate.

However, use of an antiseptic below a minimum inhibitory concentration can contribute to a rise in microbial cross-resistance to both antiseptics and antibiotics because of the survival of resistant cells within a population. This clinically dangerous phenomenon is particularly well-described for Gram-negative pathogens such as *Pseudomonas aeruginosa* and *Acinetobacter spp.* and is related to the overexpression of efflux pumps able to actively remove chlorhexidine and antibiotics outside of the bacterial cytoplasm.

Although modern antiseptics are very efficient antimicrobials, bacteria living within chronic wounds are able to survive in many cases. One of the reasons for this is the ability of microbes to form a biofilm. A biofilm is a highly organized, microbial community containing not only metabolically active but also slow-growing and dormant cells. This multi-cellular society of aggregated microorganisms is enclosed within a self-produced extracellular matrix (ECM). The matrix may account for even 90% of biofilm's dry mass and may consist of proteins, glycoproteins, polysaccharides, and extracellular DNA. The ECM provides bacteria with protection, a nutrition source, and an environment in which virulence factors or messenger molecules are interchanged rapidly and effectively. Moreover, bacteria distributed in various layers of ECM display metabolic differentiation. This phenomenon is considered an important component of observed high tolerance of biofilm against various antimicrobial agents compared to planktonic (non-adhered) cells of the same microbial strain.

The chronic wound environment itself is the second reason, explaining many cases of failed therapy. The majority of chronic wounds produce an exudate, a turbid cellular fluid which can dilute antiseptic concentration, and also binds antiseptic molecules to proteins and/or blood cells contained within the wound. Moreover, topographical irregularities and niches in the wound may be used by the microbes as a shelter from unfavorable agents. In addition, in the course of their evolutionary relationship with humans, predominant wound pathogens developed the ability to use host fibrinogen and to transform it into fibrin. These fibrin accretions form a clot with bacteria contained within it, protecting them from antimicrobials and immune responses.

At first glance, increasing the concentration of active substances in an antiseptic may appear to be a possible solution for overcoming the aforementioned issues. However, the application of higher concentrations of antiseptics leads to a cytotoxic effect on cells of the wound bed and ultimately to the inhibition of the healing process.

The problem to be solved thus refers to the provision of a possibility of increasing the antimicrobial agent's antimicrobial and/or anti-biofilm efficacy without an increase in its concentration.

Recent research hypothesized that the application of a rotating magnetic field (RMF) coupled with antimicrobials may increase the efficacy of antiseptics and/or antibiotics (e.g., Junka et al. (2018), Nature Scientific Reports, vol. 8:167, p.1-12; Ciecholewska-Jusko et al. (2022), Nature Scientific Reports, vol. 12:8836, p.1-18).

Magnetic fields are areas where magnetic forces act on moving electric charges, magnetic materials or other magnets at a distance. The fields also arise from magnets and moving electric charges. There are numerous scientific reports on the effects, both negative and positive ones, concerning the impact of magnetic fields on humans and on other organisms. The negative effects include carcinogenesis, while the positive ones include stimulation of bone repair and chronic wound healing. The biological background of these effects still needs to be elucidated. Proposed explanations focus mainly on the impact of magnetic fields on charged molecules, with a special emphasis placed on various cell membrane structures and electrically charged molecules in bacterial environments (e.g. culture media, water, or other liquids within which microbes are suspended).

The present invention refers to the use of magnetic fields for the treatment of infected biological tissues, preferably of a wound. The present description reveals that the application of the magnetic field variation has an improved impact on microbial cell death over the rotating magnetic field presented in prior art reports, especially in combination with antibacterial and/or antimicrobial agents. The invention presented herein refers to antiseptic or antibiotic agents for use in treating an infected tissue such as a wound in a subject, wherein the subject is to receive a magnetic field variation treatment at the infected tissue before, after or concurrently with the application of the agent, and wherein the magnetic field variation treatment comprises at least two phases, wherein in each phase a different type of periodic wave is applied, wherein the periodic waves differ in their periodic waveform, their frequency and/or their intensity.

Thus, the present invention provides an improved therapeutic contribution to the prior art. Taking into account the urgent clinical need for the provision of new therapeutic options effective against methicillin-resistant strains, the present invention is of utmost importance and provides a basis for new treatment options for MRSA infections. Moreover, the present invention describes a new treatment group of subjects which is considered an important route into the application of magnetic fields to fight infections caused by methicillin-resistant staphylococci.

### SUMMARY OF THE INVENTION

In a first aspect, the invention refers to an antiseptic or antibiotic agent (agent) for use in treating a wound in a subject, wherein the subject is to receive a magnetic field treatment at the wound before, after or concurrently with the application of the agent, and wherein the magnetic field treatment comprises at least two phases of periodic waves, wherein in each consecutive phase a different type of periodic wave is applied, wherein the periodic waves differ in their waveform, their frequency and/or their intensity.

In an embodiment, the invention refers to an antiseptic or antibiotic agent (agent) for use in treating a wound in a subject, wherein the subject is to receive a magnetic field treatment at the wound before, after or concurrently with the application of the agent, and wherein the magnetic field treatment comprises at least two phases of periodic waves, wherein in each phase a different type of periodic wave is applied, wherein the periodic waves differ in their waveform, their frequency and/or their intensity.

In an embodiment, the magnetic field is an alternating magnetic field.

In an embodiment, the magnetic field treatment comprises periodic waves with an amplitude range of 0.1-100 mT.

In an embodiment, the magnetic field treatment comprises periodic waves with a frequency spectrum between 0,1 and 15000 Hz and/or with a variation of the frequency. The magnetic field treatment may thus comprise in one phase periodic waves with a constant frequency and in a further phase periodic waves with increasing or decreasing frequencies. In an embodiment, one phase may comprise more than one of a periodic wave with a constant frequency or a periodic wave with increasing or decreasing frequency. In a further embodiment, the magnetic field treatment comprises in a phase simultaneous application of two or more periodic waves, wherein the two or more periodic waves differ in their frequency

In an embodiment, the periodic waveforms comprise sinusoidal waves, sawtooth waves, squared waves, triangle waves, and/or arbitrary waveforms.

In an embodiment, the at least two phases are at least two successive phases applied immediately after each other.

In an embodiment, the magnetic field treatment is applied for 0.5 to 12 hours, preferably for 0.5, 1 or 2 hours. The magnetic field treatment may be applied for 0.5 to 12 hours, preferably for 0.5, 1 or 2 hours at the wound.

The magnetic field treatment may be applied to the wound 30-60 min after the application of the agent. In other embodiments, the application of the agent is applied 30-60 min before the application of the agent.

The agent may be an antiseptic. In other embodiments, the agent may be an antibiotic. In an embodiment, the agent may be selected from the group comprising alcohols, iodine compounds, biguanides, peroxides, silver compounds, octenidine dihydrochloride, and beta-lactams, macrolides, fluoroquinolones, tetracyclines, aminoglycosides, sulfonamides, glycopeptides, oxazolidinones, lincosamides, nitroimidazoles, polymyxins, streptogramins, ansamycins, lipopeptides, and plant-based antimicrobials.

In a preferred embodiment, the wound is a wound infected with bacteria, preferably with *pseudomonas* sp. and/or *staphylococcus* sp. In an embodiment, the wound is infected with methicillin-resistant staphylococci.

In another aspect, the invention refers to an *in vitro* method of decreasing microbial load of a tissue, the method comprising applying an antibiotic agent (agent) to the tissue prior to, concurrently with, or after applying a magnetic field treatment to the tissue, wherein the magnetic field treatment comprises at least two phases, wherein in each phase a different type of periodic wave is applied, wherein the periodic waves differ in their periodic waveform, their frequency and/or their intensity.

In an embodiment, the magnetic field treatment comprises periodic waves with an amplitude range of 0.1-100 mT.

In an embodiment, the magnetic field treatment comprises periodic waves with a frequency spectrum between 0,1 and 15000 Hz and/or with a variation of the frequency. The magnetic field treatment may thus comprise in one phase periodic waves with a constant frequency and in a further phase periodic waves with increasing or decreasing frequencies. In an embodiment, one phase may comprise more than one of a periodic wave with a constant frequency or a periodic wave with increasing or decreasing frequency. In a further embodiment, the magnetic field treatment comprises in a phase simultaneous application of two or more periodic waves, wherein the two or more periodic waves differ in their frequency

In an embodiment, the periodic waveforms comprise sinusoidal waves, sawtooth waves, squared waves, triangle waves, and/or arbitrary waveforms.

In an embodiment, the magnetic field treatment is applied for 0.5 to 12 hours, preferably for 0.5, 1 or 2 hours. The magnetic field treatment may be applied for 0.5 to 12 hours, preferably for 0.5, 1 or 2 hours at the tissue.

The magnetic field treatment may be applied to the tissue 30-60 min after the application of the agent. In other embodiments, the application of the agent is applied 30-60 min before the application of the agent.

The agent may be an antiseptic. In other embodiments, the agent may be an antibiotic. In an embodiment, the agent may be selected from the group comprising alcohols, halogenated phenols, quaternary ammonium compounds, iodine compounds, biguanides, peroxides, silver compounds, and aldehydes, beta-lactams, macrolides, fluoroquinolones, tetracyclines, aminoglycosides, sulfonamides, glycopeptides, oxazolidinones, lincosamides, nitroimidazoles, polymyxins, streptogramins, ansamycins, lipopeptides and plant-based antimicrobials.

In yet another aspect, the invention refers to an *in vitro* method of enhancing microbial cell susceptibility to an antibiotic agent (agent), the method comprising applying a magnetic field treatment to the microbial cell, wherein the magnetic field treatment comprises at least two phases, wherein in each phase a different type of periodic wave is applied, wherein the periodic waves differ in their periodic waveform, their frequency and/or their intensity.

In an embodiment, the magnetic field treatment comprises periodic waves with an amplitude range of 0.1-100 mT.

In an embodiment, the magnetic field treatment comprises periodic waves with a frequency spectrum between 0,1 and 15000 Hz and/or with a variation of the frequency. The magnetic field treatment may thus comprise in one phase periodic waves with a constant frequency and in a further phase periodic waves with increasing or decreasing frequencies, and/or simultaneous application of multiple frequences or ranges of frequencies. In an embodiment, one phase may comprise more than one of a periodic wave with a constant frequency or a periodic wave with increasing or decreasing frequency. In a further embodiment, the magnetic field comprises in a phase simultaneous application of two or more periodic waves, wherein the two or more periodic waves differ in their frequency

In an embodiment, the periodic waveforms comprise sinusoidal waves, sawtooth waves, squared waves, triangle waves, and/or arbitrary waveforms.

In an embodiment, the magnetic field treatment is applied for 0.5 to 12 hours, preferably for 0.5, 1 or 2 hours. The magnetic field treatment may be applied for 0.5 to 12 hours, preferably for 0.5, 1 or 2 hours at the microbial cell.

The magnetic field treatment may be applied to the microbial cell 30-60 min after the application of the agent. In other embodiments, the application of the agent is applied 30-60 min before the application of the agent.

The agent may be an antiseptic. In other embodiments, the agent may be an antibiotic. In an embodiment, the agent may be selected from the group comprising alcohols, halogenated phenols, quaternary ammonium compounds, iodine compounds, biguanides, peroxides, silver compounds, and aldehydes, beta-lactams, macrolides, fluoroquinolones, tetracyclines, aminoglycosides, sulfonamides, glycopeptides, oxazolidinones, lincosamides, nitroimidazoles, polymyxins, streptogramins, ansamycins, lipopeptides and plant-based antimicrobials.

### BRIEF DESCRIPTION OF THE FIGURES

**Figure 1****:** CFU ratio of treated (OCT or OCT + MF (OMF)) planktonic MRSA23 and MRSA25 cell cultures. MF: 30 min/SAW/50Hz - 30min/SIN/50Hz or 30 min/SAW/0-2000 - 30 min/SAW/50Hz. OCT - octenidine, SIN - sinusoidal waveform, SAW - sawtooth waveform.
**Figure 2****: (A)** Spinning disk confocal microscope images of treated (OCT or OCT + MF) *S*. *aureus* ATCC6538 biofilms, MF: 30min/SIN/50Hz - 30 min/SAW/50Hz. **(B)** SEM image of a treated (OCT or OCT + MF) MRSA23 biofilm (MF:10min/SIN/50Hz - 10min/SAW/2000-0HZ - 10min/SIN/50Hz). OCT - octenidine, SIN - sinusoidal waveform, SAW - sawtooth waveform
**Figure 3****:** Imago testing: the number of larvae that transformed into imago forms and thus survived the MF treatment.

### DETAILED DESCRIPTION OF THE INVENTION

### Definitions

Unless defined otherwise, all technical and scientific terms used herein have the same meaning as commonly understood by one of ordinary skill in the art.

**Numerical ranges** include the numbers defining the range. All numbers are understood to encompass the midpoints of integers above and below the integer, i.e., number 2 encompasses 1.5-2.5. The number 2.5 encompasses 2.45-2.55 etc. When sample values are provided, unless otherwise specified, for example, each individually can represent an intermediate value in a range of values, and together can represent an extreme value in the range.

As used herein, the articles **"a",** "an", and "the" relate equivalently to a meaning as singular or plural unless the context dictates otherwise.

A **"biofilm"** is a sessile or bound community of microbial cells. A biofilm may develop on wounds. There, microorganisms adhere to the surface of the wound and multiply rapidly in a warm and moist environment. They are able to build a layer of mucus, the polymeric matrix, that gives them adaptive tolerance to immunity and imparts antimicrobial resistance.

**"Planktonic"** cells are non-adhered cells, here referring to microbial cells. This is in contrast to microbial cells in a biofilm.

A **"magnetic field"** can be regarded as the area around a magnet or a moving charged particle within which magnetic force is exerted. The field is produced and affected by magnets and movement of electric charges. The presence and strength of a magnetic field is denoted by "magnetic flux lines". The direction of the magnetic field is also indicated by these lines. The closer the lines, the stronger the magnetic field.

**"Alternating magnetic field"** is when the polarity changes according to the cyclic changes of the current flow direction in the field-creating electrical conductor.

**"Magnetic field variation treatment"** is a sequence of consecutive phases of magnetic field variations, characterized by distinctly different parameters, such as waveform, amplitude, frequency, and the like.

**"Rotating magnetic field"** is when the magnetic field is produced by two alternating currents or moving polarities interacting synchronously, with opposite poles rotating with synchronous speed, around a central axis.

A **"phase of periodic waves"** means a part of the applied therapeutic scheme of the applied magnetic field. Thus "phase" is interchangeable with "part", "portion", "cycle" and the like.

**"Arbitrary waveform"** is a periodic waveform of any shape.

"mT" is the abbreviation for Millitesla.

**"Antimicrobial"** means antiseptic and/or antibiotic.

**"Antimicrobial agent"** refers to an antiseptic agent and/or an antibiotic agent. An antimicrobial agent is an antibiotic agent. An antimicrobial agent is an antiseptic agent.

An **"antiseptic agent"** is an antimicrobial substance or compound that is applied to living tissue to reduce the possibility of sepsis, infection or putrefaction.

An **"antibiotic agent"** is an antimicrobial substance or compound that destroys microorganisms within the body.

**"Disinfectants"** are non-selective agents, such as bleach, which kill a wide range of microbes on non-living surfaces to prevent the spread of illness.

**"Methicillin-resistance"** refers to a state when the beta-lactam antibiotic methicillin shows no or very reduced effect against gram-positive bacteria, such as *Staphylococcus aureus.* Such bacteria are then referred to as methicillin-resistant, e.g., methicillin-resistant *Staphylococcus aureus* (MRSA).

**"Wound"** refers to a tissue infected with microbes.

**"Microbes"** refer to bacteria and fungi. In embodiments, microbes refer to bacteria.

**"Subject"** refers to a patient or living creature in need of the herein presented therapy.

### Effects of the invention

The present invention refers to a combination therapy against bacterial infections. The combination therapy combines the use of antibiotics and/or antiseptics with the exposure of the infected specimen to a magnetic field (MF). It could be shown that the susceptibility of bacterial cells to antibiotic or antiseptic agents is enhanced when the bacteria are exposed to a magnetic field. In other words, the same concentration of the agent is more efficient against bacteria when the infected specimen is exposed to a magnetic field. Or in yet other words, the activity of the agent is increased in the presence of a magnetic field towards a biofilm or planktonic bacteria. This effect has been shown to be even more increased in the case of the magnetic field variation treatment (MFVT) disclosed herein. The MIC value of the respective agent could be reduced in all tested bacterial strains if the agent had been applied together with exposure to a magnetic field. Such effect may at least partially be based on the fact that the magnetic field applied in the methods disclosed herein leads to alterations of cell integrity such as deformed cell shapes, decreased turgor or partial disruption of cell walls. Disruption of the bacterial cell wall, while not complete, allowed the antimicrobial agent to penetrate more efficiently into the cell, potentially explaining the enhanced antimicrobial action. Electron microscopic imaging provides detailed visualization of the alterations induced by the exposure to a magnetic field. Notably, microscopic analysis showed that certain parts of the cell wall experienced more damage than others, suggesting specific interaction points or a degree of heterogeneity in the cell wall structure and its interaction with MF and the agent. It could be further shown that the exposure to MF is biocompatible and as such not harmful to a cell's viability. Another positive effect of the invention is that by using the herein disclosed methods, methicillin-resistant bacteria strains can effectively be eradicated. Moreover, the combination of MFVT and an antimicrobial agent may be particularly useful in eradicating biofilms located in such areas as wound pockets, where physical obstacles limit antiseptic/antibiotic activity. In addition, in contrast to rotating magnetic fields (RMF) the MFVT applied in the present invention provides for the use of more suitable MF-generating devices, thus simplifying the therapy setting at the wound.

### Antimicrobial agents

An antimicrobial agent referred to in the present description is an agent that kills microorganisms or stops their growth. Antimicrobial agents comprise antibiotic agents, antiseptic agents, and antifungals. Preferably, antimicrobial agents used with the invention are antibiotics and antiseptics. Preferably, the antimicrobial agent is an antibiotic agent. Also preferably, the antimicrobial agent is an antiseptic agent.

### Antibiotic Agents

The antibiotic agent may be selected from the classes consisting of beta-lactam antibiotics, aminoglycosides, ansa-type antibiotics, anthraquinones, quinolones, antibiotic azoles, oxazolidinones, antibiotic glycopeptides, macrolides, antibiotic nucleosides, antibiotic peptides, antibiotic polyenes, antibiotic polyethers, antibiotic steroids, sulfonamides, tetracycline, dicarboxylic acids, antibiotic metals, oxidizing agents, substances that release free radicals and/or active oxygen, cationic antimicrobial agents, peptidyl transferases, halogens, fluoroquinolones, lincosamides, nitroimidazoles, streptogramins, ketolides and analogs and polymers thereof and naturally occurring antibiotic compounds. Preferably, antibiotic agents are selected from the classes of beta-lactams, macrolides, quinolones, tetracyclines and aminoglycosides.

Beta-lactam antibiotics include, but are not limited to, 2-(3-alanyl)clavam, 2-hydroxymethylclavam, 8-epithienamycin, acetyl-thienamycin, amoxicillin, amoxicillin sodium, amoxicillin trihydrate, amoxicillin-potassium clavulanate combination, ampicillin, ampicillin sodium, ampicillin trihydrate, ampicillin-sulbactam, apalcillin, aspoxicillin, azidocillin, azlocillin, aztreonam, bacampicillin, biapenem, carbenicillin, carbenicillin disodium, carfecillin, carindacillin, carpetimycin, cefacetril, cefaclor, cefadroxil, cefalexin, cefaloridine, cefalotin, cefamandole, cefamandole, cefapirin, cefatrizine, cefatrizine propylene glycol, cefazedone, cefazolin, cefbuperazone, cefcapene, cefcapene pivoxil hydrochloride, cefdinir, cefditoren, cefditoren pivoxil, cefepime, cefetamet, cefetamet pivoxil, cefixime, cefmenoxime, cefmetazole, cefminox, cefminox, cefmolexin, cefodizime, cefonicid, cefoperazone, ceforanide, cefoselis, cefotaxime, cefotetan, cefotiam, cefoxitin, cefozopran, cefpiramide, cefpirome, cefpodoxime, cefpodoxime proxetil, cefprozil, cefquinome, cefradine, cefroxadine, cefsulodin, ceftazidime, cefteram, cefteram pivoxil, ceftezole, ceftibuten, ceftizoxime, ceftriaxone, cefuroxime, cefuroxime axetil, cephalosporin, cephamycin, chitinovorin, ciclacillin, clavulanic acid, clometocillin, cloxacillin, cycloserine, deoxy pluracidomycin, dicloxacillin, dihydro pluracidomycin, epicillin, epithienamycin, ertapenem, faropenem, flomoxef, flucloxacillin, hetacillin, imipenem, lenampicillin, loracarbef, mecillinam, meropenem, metampicillin, meticillin, mezlocillin, moxalactam, nafcillin, northienamycin, oxacillin, panipenem, penamecillin, penicillin, phenethicillin, piperacillin, tazobactam, pivampicillin, pivcefalexin, pivmecillinam, pivmecillinam hydrochloride, pluracidomycin, propicillin, sarmoxicillin, sulbactam, sulbenicillin, talampicillin, temocillin, terconazole, thienamycin, ticarcillin and analogs, salts and derivatives thereof. Preferably, beta-lactams are selected from the group comprising penicillins, cephalosporins, monobactams, and carbapenems.

Aminoglycosides include but are not limited to, streptomycin, dihydrostreptomycin, neomycin (framycetin, paromomycin, ribostamycin), kanamycin (amikacin, arbekacin, bekanamycin, dibekacin), tobramycin (ioteprednol), spectinomycin, hygromycin B, totomycin, apramycin, nourseothricin, gentamicin (netilmicin, sisomicin, micronomicin, plazomicin, isepamicin), verdamicin, astromicin, and butirosin. Moreover, aminoglycosides include amikacin, amikacin sulfate, apramycin, arbekacin, astromicin, astromicin sulfate, bekanamycin, bluensomycin, boholmycin, butirosin B, catenulin, coumamidine gamma1, coumamidine gamma2,D,L-1-N-(alpha-hydroxy-beta-aminopropionyl)-XK-62-2, dactimicin, de-O-methyl-4-N-glycyl-KA-6606VI, de-O-methyl-KA-66061, de-O-methyl-KA-70381, destomycin A, destomycin B, di-N6',O3-demethylistamycin A, dibekacin, dibekacin sulfate, epi-formamidoylglycidylfortimicin B, epihygromycin, formimidoyl-istamycin A, formimidoyl-istamycin B, fortimicin B, fortimicin C, fortimicin D, fortimicin KE, fortimicin KF, fortimicin KG, fortimicin KG1 (stereoisomer KG1/KG2), fortimicin KG2 (stereoisomer KG1/KG2), fortimicin KG3, framycetin, framycetin sulphate, gentamicin, gentamycin sulfate, globeomycin, hybrimycin A1, hybrimycin A2, hybrimycin B1, hybrimycin B2, hybrimycin C1, hybrimycin C2, hygromycin, hygromycin B, isepamicin, isepamicin sulfate, istamycin, kanamycin, kanamycin sulphate, kasugamycin, lividomycin, marcomycin, micronomicin, micronomicin sulfate, mutamicin, myomycin, N-demethyl-7-O-demethylcelesticetin, demethylcelesticetin, methanesulfonic acid derivative of istamycin, nebramycin, nebramycin, neomycin, netilmicin, oligostatin, paromomycin, quintomycin, ribostamycin, saccharocin, seldomycin, sisomicin, sorbistin, spectinomycin, trehalosmaine, trestatin, validamycin, xylostasin, zygomycin and analogs, salts and derivatives thereof.

Ansa-type antibiotics (ansamycins) include, but are not limited to, 21-hydroxy-25-demethyl-25- 3-methylthiorifamycin, ansamitocin, atropisostreptovaricin, awamycin, halomicin, maytansine, naphthomycin, rifabutin, rifamide, rifampicin, rifamycin, rifapentine, rifaximin, rubradirin, streptovaricin, tolypomycin and analogs, salts and derivatives thereof.

Antibiotic anthraquinones include, but are not limited to, auramycin, cinerubin, ditrisarubicin, ditrisarubicin C, figaroic acid fragilomycin, minomycin, rabelomycin, rudolfomycin, sulfurmycin and analogs, salts and derivatives thereof.

Quinolones include, but are not limited to, an alkyl-methylendioxy-4(1H)-oxocinnoline-3-carboxylic acid, alatrofloxacin, cinoxacin, ciprofloxacin, ciprofloxacin hydrochloride, danofloxacin, dermofongin A, enoxacin, enrofloxacin, fleroxacin, flumequine, gatifloxacin, gemifloxacin, grepafloxacin, levofloxacin, lomefloxacin, lomefloxacin, hydrochloride, miloxacin, moxifloxacin, nadifloxacin, nalidixic acid, nifuroquine, norfloxacin, ofloxacin, orbifloxacin, oxolinic acid, pazufloxacine, pefloxacin, pefloxacin mesylate, pipemidic acid, piromidic acid, premafloxacin, rosoxacin, rufloxacin, sparfloxacin, temafloxacin, tosufloxacin, trovafloxacin and analogs, salts and derivatives thereof.

Antibiotic azoles include, but are not limited to, azanidazole, bifonazole, butoconazol, chlormidazole, chlormidazole hydrochloride, cloconazole, cloconazole monohydrochloride, clotrimazol, dimetridazole, econazole, econazole nitrate, enilconazole, fenticonazole, fenticonazole nitrate, fezatione, fluconazole, flutrimazole, isoconazole, isoconazole nitrate, itraconazole, ketoconazole, lanoconazole, metronidazole, metronidazole benzoate, miconazole, miconazole nitrate, neticonazole, nimorazole, niridazole, omoconazol, omidazole, oxiconazole, oxiconazole nitrate, propenidazole, secnidazol, sertaconazole, sertaconazole nitrate, sulconazole, sulconazole nitrate, tinidazole, tioconazole, voriconazol and analogs, salts and derivatives thereof.

Oxazolidinones comprising eperozolid, linezolid, posizolid, radezolid, ranbezolid, sutezolid, tedizolid.

Antibiotic glycopeptides include, but are not limited to, acanthomycin, actaplanin, avoparcin, balhimycin, bleomycin B (copper bleomycin), chloroorienticin, chloropolysporin, demethylvancomycin, enduracidin, galacardin, guanidylfungin, hachimycin, demethylvancomycin, N-nonanoyl-teicoplanin, phleomycin, platomycin, ristocetin, staphylocidin, talisomycin, teicoplanin, vancomycin, victomycin, xylocandin, zorbamycin and analogs, salts and derivatives thereof.

Macrolides include, but are not limited to, acetylleucomycin, acetylkitasamycin, angolamycin, azithromycin, bafilomycin, boromycin, brefeldin, carbomycin, chalcomycin, cirramycin, clarithromycin, concanamycin, deisovaleryl-niddamycin, demycinosyl-mycinamycin, Di-O-methyltiacumicidin, dirithromycin, erythromycin, erythromycin estolate, erythromycin ethyl succinate, erythromycin lactobionate, erythromycin stearate, flurithromycin, focusin, foromacidin, gamithromycin, haterumalide, haterumalide, josamycin, josamycin ropionate, juvenimycin, juvenimycin, kitasamycin, ketotiacumicin, lankavacidin, lankavamycin, leucomycin, machecin, maridomycin, megalomicin, methylleucomycin, methymycin, midecamycin, miocamycin, mycaminosyltylactone, mycinomycin, neutramycin, niddamycin, nonactin, oleandomycin, phenylacetyldeltamycin, pamamycin, picromycin, rokitamycin, rosaramicin, roxithromycin, sedecamycin, shincomycin, solithromycin, spiramycin, swalpamycin, tacrolimus, telithromycin, tiacumicin, tildipirosin, tilmicosin, treponemycin, troleandomycin, tulathromycin, tylosin, tylvalosin, venturicidin and analogs, salts and derivatives thereof.

Antibiotic nucleosides include, but are not limited to, amicetin, angustmycin, azathymidine, blasticidin S, epiroprim, flucytosine, gougerotin, mildiomycin, nikkomycin, nucleocidin, oxanosine, oxanosine, puromycin, pyrazomycin, showdomycin, sinefungin, sparsogenin, spicamycin, tunicamycin, uracil polyoxin, vengicide and analogs, salts and derivatives thereof.

Antibiotic peptides include, but are not limited to, actinomycin, aculeacin, alazopeptin, amfomycin, amythiamycin, antifungal from Zalerion arboricola, antrimycin, apid, apidaecin, aspartocin, auromomycin, bacileucin, bacillomycin, bacillopeptin, bacitracin, bagacidin, beminamycin, beta-alanyl-L-tyrosine, bottromycin, capreomycin, caspofungine, cepacidine, cerexin, cilofungin, circulin, colistin, cyclodepsipeptide, cytophagin, dactinomycin, daptomycin, decapeptide, desoxymulundocandin, echanomycin, echinocandin B, echinomycin, ecomycin, enniatin, etamycin, fabatin, ferrimycin, ferrimycin, ficellomycin, fluoronocathiacin, fusaricidin, gardimycin, gatavalin, globopeptin, glyphomycin, gramicidin, herbicolin, iomycin, iturin, iyomycin, izupeptin, janiemycin, janthinocin, jolipeptin, katanosin, killertoxin, lipopeptide antibiotic, lipopeptide from Zalerion sp., lysobactin, lysozyme, macromomycin, magainin, melittin, mersacidin, mikamycin, mureidomycin, mycoplanecin, mycosubtilin, neopeptifluorin, neoviridogrisein, netropsin, nisin, nocathiacin, nocathiacin 6-deoxyglycoside, nosiheptide, octapeptin, pacidamycin, pentadecapeptide, peptifluorin, permetin, phytoactin, phytostreptin, planothiocin, plusbacin, polcillin, polymyxins such as polymyxin antibiotic complex, polymyxin B, polymyxin B1, polymyxin F, preneocarzinostatin, quinomycin, quinupristin-dalfopristin, safracin, salmycin, salmycin, salmycin, sandramycin, saramycetin, siomycin, sperabillin, sporamycin, a streptomyces compound, subtilin, teicoplanin aglycone, telomycin, thermothiocin, thiopeptin, thiostrepton, tridecaptin, tsushimycin, tuberactinomycin, tuberactinomycin, tyrothricin, valinomycin, viomycin, virginiamycin, zervacin and analogs, salts and derivatives thereof.

In one or more embodiments, the antibiotic peptide is a naturally-occurring peptide that possesses an antibacterial and/or an antifungal activity. Such peptide can be obtained from a herbal or a vertebrate source.

Polyenes include, but are not limited to, amphotericin, amphotericin, aureofungin, ayfactin, azalomycin, blasticidin, candicidin, candicidin methyl ester, candimycin, candimycin methyl ester, chinopricin, filipin, flavofungin, fradicin, hamycin, hydropricin, levorin, lucensomycin, lucknomycin, mediocidin, mediocidin methyl ester, mepartricin, methylamphotericin, natamycin, niphimycin, nystatin, nystatin methyl ester, oxypricin, partricin, pentamycin, perimycin, pimaricin, primycin, proticin, rimocidin, sistomycosin, sorangicin, trichomycin and analogs, salts and derivatives thereof.

Polyethers include, but are not limited to, 20-deoxy-epi-narasin, 20-deoxysalinomycin, carriomycin, dianemycin, dihydrolonomycin, etheromycin, lonomycin, iso-lasalocid, lasalocid, lenoremycin, lonomycin, lysocellin, monensin, narasin, oxolonomycin, a polycyclic ether antibiotic, salinomycin and analogs, salts and derivatives thereof.

Antibiotic steroids include, but are not limited to, aminosterol, ascosteroside, cladosporide A, dihydrofusidic acid, dehydro-dihydrofusidic acid, dehydrofusidic acid, fusidic acid, squalamine and analogs, salts and derivatives thereof.

Sulfonamides include, but are not limited to, chloramine, dapsone, mafenide, phthalylsulfathiazole, succinylsulfathiazole, sulfabenzamide, sulfacetamide, sulfachlorpyridazine, sulfadiazine, sulfadiazine silver, sulfadicramide, sulfadimethoxine, sulfadoxine, sulfaguanidine, sulfalene, sulfamazone, sulfamerazine, sulfamethazine, sulfamethizole, sulfamethoxazole, sulfamethoxypyridazine, sulfamonomethoxine, sulfamoxol, sulfanilamide, sulfaperine, sulfaphenazol, sulfapyridine, sulfaquinoxaline, sulfasuccinamide, sulfathiazole, sulfathiourea, sulfatolamide, sulfatriazin, sulfisomidine, sulfisoxazole, sulfisoxazole acetyl, sulfacarbamide and analogs, salts and derivatives thereof.

Tetracyline antibiotics comprise tetracyclines including, but not limited to, aclacinomycin, akrobomycin, baumycin, bromotetracycline, cetocyclin, chlortetracycline, clomocycline, daunorubicin, demeclocycline, doxorubicin, doxorubicin hydrochloride, doxycycline, dihydrosteffimycin, demethyltetracycline, eravacycline, lymecyclin, marcellomycin, meclocycline, meclocycline sulfosalicylate, methacycline, minocycline, minocycline hydrochloride, musettamycin, omadacycline, oxytetracycline, penimepicycline, rhodirubin, rolitetracycline, rubomycin, sarecycline, serirubicin, steffimycin, tetracycline and analogs, salts and derivatives thereof.

Dicarboxylic acids, having between about 6 and about 14 carbon atoms in their carbon atom skeleton are particularly useful in the treatment of disorders of the skin and mucosal membranes that involve microbial. Suitable dicarboxylic acid moieties include, but are not limited to, adipic acid, pimelic acid, suberic acid, azelaic acid, sebacic acid, 1,11-undecanedioic acid, 1,12-dodecanedioic acid, 1,13-tridecanedioic acid and 1,14-tetradecanedioic acid. Thus, in one or more embodiments of the present invention, dicarboxylic acids, having between about 6 and about 14 carbon atoms in their carbon atom skeleton, as well as their salts and derivatives (e.g., esters, amides, mercapto-derivatives, anhydraides), are useful immunomodulators in the treatment of disorders of the skin and mucosal membranes that involve inflammation. Azelaic acid and its salts and derivatives are preferred. It has antibacterial effects on both aerobic and anaerobic organisms, particularly propionibacterium acnes and staphylococcus epidermidis, normalizes keratinization, and has a cytotoxic effect on malignant or hyperactive melanocytes. In a preferred embodiment, the dicarboxylic acid is azelaic acid in a concentration greater than 10%. Preferably, the concentration of azelaic acid is between about 10% and about 25%. In such concentrations azelaic acid is suitable for the treatment of a variety of skin disorders, such as acne, rosacea and hyperpigmentation.

In one or more embodiments, the antibiotic agent is an antibiotic metal. A number of metals ions have been shown to possess antibiotic activity, including silver, copper, zinc, mercury, tin, lead, bismutin, cadmium, chromium and ions thereof. It has been theorized that these antibiotic metal ions exert their effects by disrupting respiration and electron transport systems upon absorption into bacterial or fungal cells. Anti-microbial metal ions of silver, copper, zinc, and gold, in particular, are considered safe for in vivo use. Anti-microbial silver and silver ions, herein also called silver compounds, are particularly useful due to the fact that they are not substantially absorbed into the body.

Thus, in one or more embodiment, the antibiotic metal consists of an elemental metal, selected from the group consisting of silver, copper, zinc, mercury, tin, lead, bismutin, cadmium, chromium and gold, which is suspended in the composition as particles, microparticles, nanoparticles or colloidal particles. The antibiotic metal can further be intercalated in a chelating substrate.

In further embodiments, the antibiotic metal is ionic. The ionic antibiotic metal can be presented as an inorganic or organic salt (coupled with a counterion), an organometallic complex or an intercalate. Non-binding examples of counter inorganic and organic ions are sulfadiazine, acetate, benzoate, carbonate, iodate, iodide, lactate, laurate, nitrate, oxide, palmitate, a negatively charged protein. In preferred embodiments, the antibiotic metal salt is a silver salt, such as silver acetate, silver benzoate, silver carbonate, silver iodate, silver iodide, silver lactate, silver laurate, silver nitrate, silver oxide, silver palmitate, silver protein, and silver sulfadiazine.

In one or more embodiments, the antibiotic metal or metal ion is embedded into a substrate, such as a polymer, a mineral (such as zeolite, clay and silica).

Oxidizing agents and substances that release free radicals and/or active oxygen. In one or more embodiments, the antibiotic agent comprises strong oxidants and free radical liberating compounds, such as oxygen, peroxides, hydrogen peroxide, benzoyl peroxide, elemental halogen species, as well as oxygenated halogen species, bleaching agents (e.g., sodium, calcium or magnesium hypochloride and the like), perchlorite species, iodine, iodate, and benzoyl peroxide. Organic oxidizing agents are also included in the definition of "oxidizing agent" according to the present invention, such as quinones. Such agents possess a potent broad-spectrum activity.

In one or more embodiments the antibiotic agent is a cationic antimicrobial agent. The outermost surface of bacterial cells universally carries a net negative charge, making them sensitive to cationic substances. Examples of cationic antibiotic agents include: quaternary ammonium compounds (QAC's)-QAC's are surfactants, generally containing one quaternary nitrogen associated with at least one major hydrophobic moiety; alkyltrimethyl ammonium bromides are mixtures of where the alkyl group is between 8 and 18 carbons long, such as cetrimide (tetradecyltrimethylammonium bromide); benzalkonium chloride, which is a mixture of n-alkyldimethylbenzyl ammonium chloride where the alkyl groups (the hydrophobic moiety) can be of variable length; dialkylmethyl ammonium halides; dialkylbenzyl ammonium halides; and QAC dimmers, which bear bipolar positive charges in conjunction with interstitial hydrophobic regions.

Peptidyl transferases including, but not limited to amphenicols such as chloramphenicol, azidamfenicol, thiamphenicol, florfenicol and pleuromutilins such as azamulin, lefamulin, retapamulin, tiamulin, valnemulin.

Halogens include, but are not limited to, chlorine, iodine, fluorine, and bromine.

Fluoroquinolones include but are not limited to enoxacin, norfloxacin, ciprofloxacin, ofloxacin, levofloxacin, moxifloxacin, sitafloxacin, nadifloxacin, lomefloxacin, and delafloxacin, and analogs, salts and derivatives thereof.

Lincosamides include but are not limited to lincomycin, clindamycin, and pirlimycin and analogs, salts and derivatives thereof.

Nitroimidazoles include but are not limited to metronidazole, tinidazole, ornidazole, fexinidazole, dimetridazol, carnidazol, ronidazol, benznidazol, pretomanide, delamanide, and analogs, salts and derivatives thereof.

Streptogramins comprising pristinamycin (IA, IIA, IIB), NXL103 (Flopristin, Linopristin), quinupristin, dalfopristin, streptogramin A, streptogramin B, virginiamycin (S1), and analogs, salts and derivatives thereof.

Ketolides, including, telithromycin, cethromycin, solithromycin.

In one or more embodiments, the antibiotic agent is a naturally occurring antibiotic compound. As used herein, the term "naturally-occurring antibiotic agent" includes all antibiotic that are obtained, derived or extracted from plant or vertebrate sources. Non-limiting examples of families of naturally-occurring antibiotic agents include phenol, resorcinol, antibiotic aminoglycosides, anamycin, quinines, anthraquinones, antibiotic glycopeptides, azoles, macrolides, avilamycin, agropyrene, cnicin, aucubin antibioticsaponin fractions, berberine (isoquinoline alkaloid), arctiopicrin (sesquiterpene lactone), lupulone, humulone (bitter acids), allicin, hyperforin, echinacoside, coniosetin, tetramic acid, imanine and novoimanine.

Ciclopirox and ciclopiroxolamine possess fungicidal, fungistatic and sporicidal activity. They are active against a broad spectrum of dermatophytes, yeasts, moulds and other fungi, such as trichophyton species, microsporum species, epidermophyton species and yeasts (candida albicans, candida glabrata, other candida species and cryptococcus neoformans). Some aspergillus species are sensitive to ciclopirox as are some penicillium. Likewise, ciclopirox is effective against many gram-positive and gram-negative bacteria (e.g., escherichia coli, proteus mirabilis, pseudomonas aeruginosa, staphylococcus and streptococcus species), as well as mycoplasma species, trichomonas vaginalis and actinomyces.

Yet, in one or more embodiment, the antibiotic is a non-classified antibiotic agent, including, without limitation, aabomycin, acetomycin, acetoxycycloheximide, acetylnanaomycin, an actinoplanes sp. Compound, actinopyrone, aflastatin, albacarcin, albacarcin, albofungin, albofungin, alisamycin, alpha-R,S-methoxycarbonylbenzylmonate, altromycin, amicetin, amycin, amycin demanoyl compound, amycine, amycomycin, anandimycin, anisomycin, anthramycin, anti-syphilis imune substance, anti-tuberculosis imune substance, antibiotic from Eschericia coli, antibiotics from Streptomyces refuineus, anticapsin, antimycin, aplasmomycin, aranorosin, aranorosinol, arugomycin, ascofuranone, ascomycin, ascosin, Aspergillus flavus antibiotic, asukamycin, aurantinin, an Aureolic acid antibiotic substance, aurodox, avilamycin, azidamfenicol, azidimycin, bacillaene, a Bacillus larvae antibiotic, bactobolin, benanomycin, benzanthrin, benzylmonate, bicozamycin, bravomicin, brodimoprim, butalactin, calcimycin, calvatic acid, candiplanecin, carumonam, carzinophilin, celesticetin, cepacin, cerulenin, cervinomycin, chartreusin, chloramphenicol, chloramphenicol palmitate, chloramphenicol succinate sodium, chlorflavonin, chlorobiocin, chlorocarcin, chromomycin, ciclopirox, ciclopirox olamine, citreamicin, cladosporin, clazamycin, clecarmycin, clindamycin, coliformin, collinomycin, copiamycin, corallopyronin, corynecandin, coumermycin, culpin, cuprimyxin, cyclamidomycin, cycloheximide, dactylomycin, danomycin, danubomycin, delaminomycin, demethoxyrapamycin, demethyiscytophycin, dermadin, desdamethine, dexylosyl-benanomycin, pseudoaglycone, dihydromocimycin, dihydronancimycin, diumycin, dnacin, dorrigocin, dynemycin, dynemycin triacetate, ecteinascidin, efrotomycin, endomycin, ensanchomycin, equisetin, ericamycin, esperamicin, ethylmonate, eveminomicin, feldamycin, flambamycin, flavensomycin, florfenicol, fluvomycin, fosfomycin, fosfonochlorin, fredericamycin, frenolicin, fumagillin, fumifungin, funginon, fusacandin, fusafungin, gelbecidine, glidobactin, grahamimycin, granaticin, griseofulvin, griseoviridin, grisonomycin, hayumicin, hayumicin, hazymicin, hedamycin, heneicomycin, heptelicid acid, holomycin, humidin, isohematinic acid, kamatakin, kazusamycin, kristenin, L-dihydrophenylalanine, a L-isoleucyl-L-2-amino-4-(4'-amino-2', 5'-cyclohexadienyl) derivative, lanomycin, leinamycin, leptomycin, libanomycin, lincomycin, lomofungin, lysolipin, magnesidin, manumycin, melanomycin, methoxycarbonylmethylmonate, methoxycarbonylethylmonate, methoxycarbonylphenylmonate, methyl pseudomonate, methylmonate, microcin, mitomalcin, mocimycin, moenomycin, monoacetyl cladosporin, monomethyl cladosporin, mupirocin, mupirocin calcium, mycobacidin, myriocin, myxopyronin, pseudoaglycone, nanaomycin, nancimycin, nargenicin, neocarcinostatin, neoenactin, neothramycin, nifurtoinol, nocardicin, nogalamycin, novobiocin, octylmonate, olivomycin, orthosomycin, oudemansin, oxirapentyn, oxoglaucine methiodide, pactacin, pactamycin, papulacandin, paulomycin, phaeoramularia fungicide, phenelfamycin, phenyl, cerulenin, phenylmonate, pholipomycin, pirlimycin, pleuromutilin, a polylactone derivative, polynitroxin, polyoxin, porfiromycin, pradimicin, prenomycin, prop-2-enylmonate, protomycin, pseudomonas antibiotic, pseudomonic acid, purpuromycin, pyrinodemin, pyrrolnitrin, pyrrolomycin, amino, chloro pentenedioic acid, rapamycin, rebeccamycin, resistomycin, reuterin, reveromycin, rhizocticin, roridin, rubiflavin, naphthyridinomycin, saframycin, saphenamycin, sarkomycin, sarkomycin, sclopularin, selenomycin, siccanin, spartanamicin, spectinomycin, spongistatin, stravidin, streptolydigin, streptomyces arenae antibiotic complex, streptonigrin, streptothricins, streptovitacin, streptozotocine, a strobilurin derivative, stubomycin, sulfamethoxazol-trimethoprim, sakamycin, tejeramycin, terpentecin, tetrocarcin, thermorubin, thermozymocidin, thiamphenicol, thioaurin, thiolutin, thiomarinol, thiomarinol, tirandamycin, tolytoxin, trichodermin, trienomycin, trimethoprim, trioxacarcin, tyrissamycin, umbrinomycin, unphenelfamycin, urauchimycin, usnic acid, uredolysin, variotin, vermisporin, verrucarin and analogs, salts and derivatives thereof.

Mixtures of these antibiotic agents may also be employed according to the present invention.

Antibiotic agents used in the present invention may be applied topically, orally or intravenously.

In preferred embodiments, an antibiotic agent for topical use is selected from the group comprising bacitracin, neomycin, polymyxin B, mupirocin, nitrofurazone, and fusidic acid. In preferred embodiments, an antibiotic agent for topical use is selected from the group consisting of bacitracin, neomycin, polymyxin B, mupirocin, nitrofurazone, and fusidic acid.

In preferred embodiments, an antibiotic agent for oral use is selected from the group comprising amoxicillin-clavulanate, cephalexin, clindamycin, dicloxacillin, doxycycline, and trimethoprim-sulfamethoxazole. In preferred embodiments, an antibiotic agent for oral use is selected from the group consisting of amoxicillin-clavulanate, cephalexin, clindamycin, dicloxacillin, doxycycline, and trimethoprim-sulfamethoxazole.

In preferred embodiments, an antibiotic agent for intra venous use is selected from the group comprising cephalosporins, penicillin, fluoroquinolones, nitroimidazoles, glycopeptides, oxazolidinone, and carbapenems. In preferred embodiments, an antibiotic agent for intra venous use is selected from the group consisting of cephalosporins, penicillin, fluoroquinolones, nitroimidazoles, glycopeptides, oxazolidinone, and carbapenems.

### Antiseptic Agents

Antiseptic agents according to the present invention include, but are not limited to, alcohols, diguanides comprising polyhexanide, iodines, octenidine dihydrochloride, peroxides, phenols, quaternary ammonium compounds, quinolines, 4-hexylresorcinol, sodium hypochlorite (NaOCI), silver nanoparticles, derivatives thereof or mixtures thereof.

Alcohols include, but are not limited to, ethyl alcohol, n-propanol such as isopropanol, isopropyl alcohol, and methanol.

Biguanides, also called Diguanides, include, but are not limited to, chlorhexidine, chlorhexidine salts, and analogs and derivatives, such as chlorhexidine acetate, chlorhexidine gluconate and chlorhexidine hydrochloride, picloxydine, alexidine and polihexanide, and polyhexanide (polyhexamethylene biguanide, PHMB).

Polyhexanide (polyhexamethylene biguanide, PHMB) is a polymer used as a disinfectant and antiseptic. Products containing PHMB are used for inter-operative irrigation, pre- and post-surgery skin and mucous membrane disinfection, post-operative dressings, surgical and non-surgical wound dressings, surgical bath/hydrotherapy, chronic wounds like diabetic foot ulcer and burn wound management, routine antisepsis during minor incisions, catheterization, first aid, surface disinfection, and linen disinfection.

Iodine compounds comprise polyvinylpyrrolidone-iodine (povidone-iodine (PVP-I)), Lugol's iodine, hydrogen iodide, iodine halides, iodine oxides and oxoacids, and analogs, salts and derivatives thereof. Moreover, compounds containing iodine are alcohol-iodine solution, an aqueous solution of iodine, and iodophors.

A preferred antiseptic used in the present invention is octenidine dihydrochloride, which is often used as a chlorhexidine substitute.

Peroxides include, but are not limited to, hydrogen peroxide and benzoyl peroxide.

Antiseptics comprise aromatic organic compounds based on acridine, such as ethactidine lactate.

Phenols and halogenated phenols include, but are not limited to, phenol itself, thymol and cresol, phenolic compounds such as chlorocresol, chloroxylenol, fentichlore, triclosan, hexachlorophene (bisphenol), 2-phenylphenol-water solutions.

Quaternary ammonium compounds (quat salts) include but is not limited to benzalkoniumchloride/lidocaine, certimide, cetylalkoniumchlorid, cetylpyridiniumchlorid, cetyltrimethylammoniumbromid, denatoniumbenzoat, dimethyldioctadecylammoniumchlorid, tetramethylammoniumhydroxid (TMAH), tetrabutylammoniumhydroxid (TBAH), paraquat, polyquaternium-7, tetradecyltrimethylammoniumoxalat, 3-chlor-2-hydroxypropyl-N,N,N-trimethylammoniumchlorid.

Quinolines such as hydroxyquinolone, dequaliniumchloride, and chlorquinaldol.

Aldehydes include, but are not limited to, formaldehyde, ortho-phtalaldehyde (OPA) and glutaraldehyde.

In preferred embodiments the antiseptic agent is applied topically in form of a spray, cream, ointment, gel, soaked pad, wipes, liquid solutions powders and/or vapor/steam.

In preferred embodiments, the antiseptic agent is selected from the group comprising alcohols, iodine compounds, octenidine dihydrochloride, sodium hypochlorite (NaOCI), silver nanoparticles and polyhexanide and mixtures thereof. In even more preferred embodiments, the antiseptic agent is selected from the group consisting of octenidine dihydrochloride, povidone-iodine, polyhexanide, sodium hypochlorite, and silver nanoparticles. Most preferably, the antiseptic agent is octenidine dihydrochloride and/or polyhexanide.

### Antifungals/antimycotics

Antifungals comprise, but are not limited to, polyenes comprising amphotericin B, candicin, filipin, hamycin, natamycin, nystatin, rimocidin; azoles comprising imidazoles, triazoles, thiazoles; allylamines, echinocandins, and triterpenoids.

### Minimum Inhibitory Concentration (MIC) and Sub-MIC

A minimum inhibitory concentration (MIC) is defined as the lowest concentration of a chemical (antimicrobial agent, here the antibacterial or antiseptic agent), which prevents visible *in vitro* growth of bacteria.

The MIC is determined by preparing a dilution series of the chemical, adding agar or broth, then inoculating with the respective bacteria, and incubating at a suitable temperature. The value obtained is largely dependent on the susceptibility of the microorganism and the antimicrobial potency of the chemical. Thus, the lower the MIC, the more potent the antimicrobial.

Accurate and precise usage of antimicrobial agents is important in the context of multidrug-resistant bacteria. Bacteria have been gaining resistance to antimicrobial agents they were previously susceptible to. Usage of incompatible levels of antimicrobials provides the selective pressure that has driven the direction and evolution of resistance of bacterial pathogens. This has been seen at sub-MIC levels of antibiotics. As such, it is increasingly important to determine the MIC in order to make the best choice in prescribing antimicrobials.

However, sub-MIC levels may be used to study the effect of the applied magnetic field. Without being bound to a theory, it is assumed that if there is a synergistic effect bacteria would be killed when the respective antimicrobial agent is applied together with the magnetic field treatment disclosed herein, while neither the antimicrobial agent nor the magnetic field alone show such effect. Such synergism could be identified with the methods disclosed herein.

### Bacteria

The present methods are suitable against any kind of bacteria. The bacteria may thus comprise gram-positive and gram-negative bacteria.

The term "bacteria" refers to bacteria associated with an infection and/or wound. Alternatively, the bacteria may be associated with infection of a tissue. Particular non-limiting examples of bacteria associated with wounds and infection include, without limitation, bacteria such as *Pseudomonas* sp. (e.g., *Pseudomonas aerugenosa*); *Staphylococcus* sp. (e.g., *Staphylococcus aureus, Staphylococcus epidermidis,* or *Staphylococcus saprophyticus*); group A and B streptococci (for example, *Streptococcus pyogenes, Streptococcus pneumoniae, Streptococcus mutans* or *Streptococcus agalactiae*), and a variety of gram-positive aerobic and anaerobic bacteria.

Others include, without limitation, *Acetinobacter anitratus, Acinetobacter baumannii, Actinoinyces* sp., *Bacillus* (for example, *Bacillus anthracis*), *Borderella perrussis, Borrelia burgdorferi, Brucella* (for example, *Brucella abortus, Brucella melitensis,* or *Brucella suis*), *Campylobacter* sp. (for example, *Campylobacterjejuni* or *Cainpylobacter fetus*), *Clostridium* sp. (for example, *Clostridium perfringens, Clostridium tetanus, Clostridium botulinum), Chlamydia* (for example, *Chlamydia psittaci, Chlamydia trachomatis,* or *Chlamydia pneumoniae*), *Clostridium difficile, Cronobacter sakazakii, Corynebacteria* sp.(for example, *Corynebacterium diptheriae*), *Enterococcus* (for example, *Enterobacter clocae, Enterococcus faecalis,* or *Enterococcus faecium*), *Escherichia* sp. (e.g., *Escherichia coli*), *Francisella tularensis, Helicobacter pylori, Haemophilus influenzae, Klebsiella pneumonia, Klebsiella oxytoca, Listeria* (for example, *Legionella pneumophila, Leptospira interrogans, Listeria monocytogenes*), *Micrococcus* sp. (e.g., *Micrococcus luteus*), *Microbacterium leprae, Mycobacterium* sp. (for example, *Mycobacterium tuberculosis, Mycobacterium ulcerans*), *Mucor* sp. (e.g., *M. plumbeus, M. racemosus*), *Mycoplasma pneumoniae, Neisseria* species (for example, *Neisseria meningitidis,* or *Neisseria gonorrhoeae*), *Nocardia* sp., *Proteus mirabilis, Pseudomonas* (for example, *Pseudomona aeruginosa* or *Pseudomonas mallei*)*, Proteus* sp. (e.g., *Proteus mirabilis*), *Rickettsia* (for example, *Rickettsia ricketsii, Rickettsia prowazekii* or *Rickettsia akari), Shigella* sp., *Serratia marcescens, Salmonella* sp., *Treponema pallidum, Ureaplasma urealyticum, Vibrio cholera, Yersinia* species (for example, *Yersinia pestis, Yersinia pseudoruberculosis,* or *Yersinia enterocolitica*), and the like.

Preferably, the bacteria is a *Staphylococcus* sp. More preferably, *Staphylococcus aureus.* In an embodiment, the *Staphylococcus aureus* is methicillin resistant. Thus, wound infection or tissue infection with *Staphylococcus* sp, preferably *Staphylococcus aureus* and/or methicillin-resistant *Staphylococcus aureus* may successfully be treated with the herein disclosed methods.

In another embodiment the bacteria is *Pseudomonas aeruginosa.* Thus, wound infection or tissue infection with *Pseudomonas aeruginosa* may successfully be treated with the herein disclosed methods.

The bacteria may be present within and/or on the wound. The bacteria may be planktonic bacteria or present in a biofilm.

### Magnetic field variation treatment (MFVT)

Magnetic fields (like other force fields) can be thought of as invisible areas around magnets or moving charges. The fields can act on other magnets or moving charges at a distance (without physical contact) and pull them or push them. A magnetic field can be visualized by assigning tiny arrows to each point in space. The size of the arrows corresponds to the magnitude of the field, and their pointy ends show towards the direction of the field.

Magnetic fields may differ based on the way they are generated and can exhibit unique characteristics. The present invention refers to a magnetic field variation treatment (MFVT), comprising of two or more phases governed by distinctly different parameters. Prior art in the field is based on rotating magnetic fields (RMF). In RMF, the orientation in space of the magnetic field vector is constantly changing (rotating), but the amplitude remains basically constant, while in MFVT the orientation remains constant, while the amplitude and direction is constantly changing. Additionally, in MFVT the magnetic field vector passes through zero, while in the RMF, this is not the case. In other words, MFVT is linearly polarized, while RMF is usually circularly or elliptically polarized. Most importantly, MFVT contains consecutive phases defined by different parameters, such as waveform, frequency, intensity, and/or variations of the above. Contrarily, RMF consists of one single phase with fixed parameters (sinusoidal waveform, single frequency).

In addition, the use of MFVT is advantageous over RMF in terms of practicability and ease-of-use. MFVT-generating devices are simpler and less expensive to build compared to RMF-generating devices, and they are much more flexible in design choices for application to the human body.

A description of a MFVT-generating device is found, for example, in WO 2022/0214195 A1.

The experimental setup of the present invention consists of a magnetic field generator with drivers and coils. Each driver is controlled by a channel-specific sub-controller, which includes a pulse-width modulation generator. Based on the generator configuration, the following magnetic field waveforms can be generated: sinusoidal, square, sawtooth, triangle, pulse and arbitrary. Combinations of these waveforms can be used to develop a specific therapy program to best address different bacterial strains.

An electronic switching circuit is connected to the sub-controller, receives the generated signals, and is configured accordingly to generate electric current. The current drives the coils and generates the specified waveforms at the specified parameters.

It is noted that MFVT has no effect on the viability of the host's cells, when applied to a potential host for bacteria (see, Example 3).

### Subject

The herein disclosed methods are intended for use in treating a wound in a subject. As used herein, the term "subject" refers to any living individual, preferably an animal, preferably a mammal, including, but not limited to, humans, non-human primates, rodents, and the like, which is to be the recipient of the herein disclosed treatment. More preferably, the subject is a human, a dog, a cat, a horse, a rat, a rabbit, a sheep, a cow, a pig. The terms "subject" and "patient" may be used interchangeably in reference to a human subject.

### Wound

The herein disclosed methods are intended for use in treating a wound in a subject. The term "wound" as used herein can refer to a chronic, acute, traumatic, subacute, and dehisced wounds, partial-thickness burns, ulcers (such as diabetic, pressure, or venous insufficiency ulcers), flaps, grafts, and fistulas, for example. The wound may be located on or in the skin of the subject. Alternatively, the wound is located within the body of the subject. A wound located within the body of a subject may be an infected tissue after surgery. The wound is an infected wound, wherein the term infected refers to the settlement of microbes, preferably bacteria within and/or on the wound. The wound may be an open or closed wound.

### Tissue

As used herein, the term "tissue" may be interchangeably used with the terms "tissue site" and "target tissue", "biological specimen" and broadly refers to any biological tissue outside a subject's body. The tissue may be a transplant. The tissue may be bone tissue, adipose tissue, muscle tissue, neural tissue, dermal tissue, vascular tissue, soft tissue, connective tissue, cartilage, tendons, ligaments, and/or the like. The term "tissue " as used herein can also refer to a surrounding tissue area(s) and/or areas of tissue that are not necessarily infected (wounded), but include tissue that would benefit from a treatment according to the herein disclosed methods. In addition, tissue may also refer to stem cells used to treat a wound and skin transplants.

### The agent for use in treating a wound - Combination therapy

The present invention refers to an antimicrobial agent for use in treating a wound in a subject, wherein the subject is to receive a magnetic field treatment at the wound. The subject thus receives a combination therapy.

The wound may be infected with any of the herein disclosed microbes, preferably bacteria. The antimicrobial agent comprises any of the herein disclosed agents, preferably selected from the group comprising alcohols, halogenated phenols, quaternary ammonium compounds, iodine compounds, biguanides, peroxides, silver compounds, and aldehydes, beta-lactams, macrolides, fluoroquinolones, tetracyclines, aminoglycosides, sulfonamides, glycopeptides, oxazolidinones, lincosamides, nitroimidazoles, polymyxins, streptogramins, ansamycins, lipopeptides and plant-based antimicrobials, or mixtures thereof.

The magnetic field treatment applied at the wound site is based on magnetic field variation. The magnetic field variation treatment comprises at least two phases of periodic waves, wherein in each consecutive phase a different type of periodic wave is applied, i.e. the periodic waves differ between the phases in their waveform, their frequency, and/or their intensity.

The applied magnetic field comprises at least two phases of periodic waves. At least two phases of periodic waves means two or more phases are applied. This means two, three, four, five, six, seven, eight, nine, ten or more phases can be applied. If more than two phases are applied, the periodic waves differ in that the ones of the consecutive phases differ from each other, i.e., the periodic wave before and the periodic wave after a certain periodic wave differ from the middle phase periodic wave. Non-consecutive phases may have the same parameters. Preferably, each periodic wave in the at least two phases differ from the periodic waves in other applied phases. Within the applied magnetic field treatment, the at least two successive phases are applied immediately after each other.

The waveforms used in the present invention comprise square, triangle, sinusoidal, sawtooth, pulsed or arbitrary waveforms, preferably square, triangle, sinusoidal, sawtooth waveforms. "Sinusoidal" is interchangeably used with "sinus".

The periodic waves may have an amplitude range of 0.1-100 mT. The amplitude may thus be 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95 or 100 mT.

A periodic wave may comprise a frequency spectrum of between 0.1 and 15000 Hz. A periodic wave may have a frequency of between 0.1 and 15000 Hz. A periodic wave may thus have a frequency of 0.1, 0.2, 0.3, 0.4, 0.5, 0.6, 0.7, 0.8, 0.9, 1, 5, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5500, 6000, 6500, 7000, 7500, 8000, 8500, 9000, 9500, 10000, 11000, 12000, 13000, 14000, or 15000 Hz. The applied magnetic field may have a frequency in the range of 0.1-50, 50-100, 100-500, 500-1000, 500-2000, 500-3000, 1000-3000, 3000-5000, 5000-10000, 10000-15000.

The frequency of a periodic wave may be constant in one phase and may be increasing or decreasing, and/or applied simultaneously with multiple frequences or ranges in another phase of the treatment. A periodic wave with an increasing frequency means that the frequency is 0 Hz or any other starting frequency at a first point and is increased up to an ending frequency. A phase of the treatment may begin with the starting frequency and end with the increased end frequency, wherein within the phase the frequency is increased. An exemplary periodic wave starts at 0 Hz and the frequency is increasing up to 5000 Hz. A periodic wave with a decreasing frequency means that the frequency is 15000 Hz or any other starting frequency at a first point and is decreased down to an ending frequency, optionally 0 Hz. A phase of the treatment may begin with the starting frequency and end with the decreased end frequency, wherein within the phase the frequency is decreased. An exemplary periodic wave starts at 5000 Hz and the frequency is decreasing down to 0 Hz.

An exemplary magnetic field treatment comprises in a first phase a sinus wave at 50 Hz and in a second phase a sinus wave with a frequency of 5000 Hz. Another exemplary magnetic field treatment comprises in a first phase a sinus wave at 50 Hz and in a second phase a sawtooth wave with a frequency of 50 Hz. Another exemplary magnetic field treatment comprises in a first phase a sinus wave at 50 Hz and in a second phase a sawtooth wave with a frequency of 50 Hz. Another exemplary magnetic field treatment comprises in a first phase a sinus wave at 50 Hz and in a second phase a sinus wave with an increasing frequency of from 10 to 1000 Hz. Another exemplary magnetic field treatment comprises in a first phase a sinus wave at 50 Hz and in a second phase a sinus wave with a decreasing frequency of from 1000 to 10 Hz. Another exemplary magnetic field treatment comprises in a first phase a triangle wave at 15000 Hz and in a second phase a sinus wave with a frequency of 15 Hz. Another exemplary magnetic field treatment comprises in a first phase a sinus wave at 50 Hz, in a second phase a sinus wave with a frequency of 5000 Hz and in a third phase a sinus wave at 50 Hz. Another exemplary magnetic field treatment comprises in a first phase a sinus wave at 200 Hz, in a second phase a sinus wave with an increasing frequency of from 10 to 1000 Hz and in a third phase a sawtooth wave at 50 Hz. Any combination of the above referenced periodic wave features is possible.

The frequency, intensity and/or waveform may be selected based on the microbes present at or in the wound.

The magnetic field treatment is applied for 0.5 to 12 hours. The treatment may preferably be applied for 10, 15, 20, 25, 30, 35, 40, 45, 50, 55, 60, 70, 80, 90, 100 minutes, or 1, 2, 3, 4, 5, 6, 7, 8, 9, 10, 11, or 12 hours. Preferably, the magnetic field treatment is applied for 0,5-2 hours. If necessary, the antiseptic and magnetic field treatment may be applied in multiple cycles. In an embodiment, the antiseptic and magnetic field treatment may be applied in multiple cycles over multiple days, weeks, or months.

The magnetic field treatment may be applied before, concurrently with, or after the application of the antimicrobial agent. The magnetic field may be applied 10-120, preferably 30-60 minutes after the antimicrobial agent has been applied. The antimicrobial incubation time may be dependent on the route of administration and/or the type of antimicrobial used. Thus, incubation time of oral antibiotics may differ from an antiseptic applied locally at or on the wound. The magnetic field may be applied and completed before the antimicrobial agent is applied. Alternatively, the magnetic field treatment is applied concurrently with the agent, i.e, the agent is applied and immediately thereafter, the magnetic field is applied.

### Methods of Treatment

The disclosure also provides a method of treating a wound in a subject in need thereof.

Therefore, the disclosure provides a method of treating a wound in a patient in need thereof, wherein the method comprises applying a magnetic field at the wound, wherein the magnetic field comprises at least two phases of periodic waves, wherein in each consecutive phase a different type of periodic wave is applied, wherein the periodic waves differ in their periodic waveform, their frequency and/or their intensity.

In an embodiment, the disclosure provides a method of treating a wound in a patient in need thereof, wherein the method comprises applying a magnetic field treatment at the wound, wherein the magnetic field treatment comprises at least two phases of periodic waves, wherein in each phase a different type of periodic wave is applied, wherein the periodic waves differ in their periodic waveform, their frequency and/or their intensity.

Preferably, the method comprises before, concurrently with or after the application of the magnetic field treatment, administering to the patient an antiseptic or antibiotic agent.

Therefore, provided herein is a method of treating a wound in a patient in need thereof, wherein the method comprises administering to the subject an antiseptic or antibiotic agent and applying a magnetic field treatment at the wound, wherein the magnetic field treatment comprises at least two phases, wherein in each consecutive phase a different type of periodic wave is applied, wherein the periodic waves differ in their periodic waveform, their frequency and/or their intensity.

In an embodiment, the invention refers to a method of treating a wound in a patient in need thereof, wherein the method comprises administering to the subject an antiseptic or antibiotic agent and applying a magnetic field treatment at the wound, wherein the magnetic field treatment comprises at least two phases, wherein in each phase a different type of periodic wave is applied, wherein the periodic waves differ in their periodic waveform, their frequency and/or their intensity.

In an embodiment, the antibiotic agent is administered orally, intravenously, or topically at and/or on the wound.

In an embodiment, the antiseptic agent is administered topically on or at the wound.

In another embodiment, the antibiotic or antiseptic agent is administered before, concurrently with or after the application of the magnetic field treatment.

The waveforms used in the present invention comprise squared, triangle, sinusoidal, sawtooth, pulsed or arbitrary waveforms, preferably squared, triangle, sinusoidal, sawtooth waveforms. "Sinusoidal" is interchangeably used with "sinus";

The periodic waves may have an amplitude range of 0.1-100 mT. The amplitude may thus be 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95 or 100 mT.

A periodic wave may comprise a frequency spectrum of between 0.1 and 15000 Hz. A periodic wave may have a frequency of between 0.1 and 15000 Hz. A periodic wave may thus have a frequency of 0.1, 0.2, 0.3, 0.4, 0.5, 0.6, 0.7, 0.8, 0.9, 1, 5, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5500, 6000, 6500, 7000, 7500, 8000, 8500, 9000, 9500, 10000, 11000, 12000, 13000, 14000, or 15000 Hz. The applied magnetic field may have a frequency in the range of 0.1-50, 50-100, 100-500, 500-1000, 500-2000, 500-3000, 1000-3000, 3000-5000, 5000-10000, 10000-15000.

The frequency of a periodic wave may be constant in one phase and may be increasing or decreasing, and/or applied simultaneously with multiple frequences or ranges of frequencies in another phase of the treatment. A periodic wave with an increasing frequency means that the frequency is 0 Hz or any other starting frequency at a first point and is increased up to an ending frequency. A phase of the treatment may begin with the starting frequency and end with the increased end frequency, wherein within the phase the frequency is increased. An exemplary periodic wave starts at 0 Hz and the frequency is increasing up to 5000 Hz. A periodic wave with a decreasing frequency means that the frequency is 15000 Hz or any other starting frequency at a first point and is decreased down to an ending frequency, optionally 0 Hz. A phase of the treatment may begin with the starting frequency and end with the decreased end frequency, wherein within the phase the frequency is decreased. An exemplary periodic wave starts at 5000 Hz and the frequency is decreasing down to 0 Hz.

An exemplary magnetic field treatment comprises in a first phase a sinus wave at 50 Hz and in a second phase a sinus wave with a frequency of 5000 Hz. Another exemplary magnetic field treatment comprises in a first phase a sinus wave at 50 Hz and in a second phase a sawtooth wave with a frequency of 50 Hz. Another exemplary magnetic field treatment comprises in a first phase a sinus wave at 50 Hz and in a second phase a sawtooth wave with a frequency of 50 Hz. Another exemplary magnetic field treatment comprises in a first phase a sinus wave at 50 Hz and in a second phase a sinus wave with an increasing frequency of from 10 to 1000 Hz. Another exemplary magnetic field treatment comprises in a first phase a sinus wave at 50 Hz and in a second phase a sinus wave with a decreasing frequency of from 1000 to 10 Hz. Another exemplary magnetic field treatment comprises in a first phase a triangle wave at 15000 Hz and in a second phase a sinus wave with a frequency of 15 Hz. Any combination of the above referenced magnetic field features is possible.

The frequency, intensity and/or waveform may be selected based on the microbes present at or in the wound.

The magnetic field is applied for 0.5 to 12 hours. The treatment may preferably be applied for 10, 15, 20, 25, 30, 35, 40, 45, 50, 55, 60, 70, 80, 90, 100 minutes, or 1, 2, 3, 4, 5, 6, 7, 8, 9, 10, 11, or 12 hours. Preferably, the magnetic field treatment is applied for 0,5-2 hours. If necessary, the antiseptic and magnetic field treatment may be applied in multiple cycles.

The magnetic field may be applied before, concurrently with, or after the application of the antimicrobial agent. The magnetic field may be applied 10-120, preferably 30-60 minutes after the antimicrobial agent has been applied. The antimicrobial incubation time may be dependent on the route of administration and/or the type of antimicrobial used. Thus, incubation time of oral antibiotics may differ from an antiseptic applied locally at or on the wound. The magnetic field may be applied and completed before the antimicrobial agent is applied. Alternatively, the magnetic field is applied concurrently with the agent, i.e, the agent is applied and immediately thereafter, the magnetic field is applied.

The method may further comprise identifying a wound, such as an infected wound. The method may further comprise indicating the use of the disclosed magnetic field treatment for the treatment of the identified wound on the subject in need of such treatment.

The method may further comprise placing a treatment coil on the body of a subject at the site of an identified wound and applying the disclosed magnetic field treatment to said wound via said treatment coil for the treatment of the identified wound.

Additionally, the disclosure provides a method of accelerating and/ or promoting wound healing in a subject in need thereof, the method comprising applying magnetic field treatment at the wound, wherein the magnetic field treatment comprises at least two phases, wherein in each consecutive phase a different type of periodic wave is applied, wherein the periodic waves differ in their periodic waveform, their frequency and/or their intensity. Preferably, the method comprises the administration or application of an antibiotic or antiseptic agent before, concurrently with or after the application of the magnetic field application, administering to the subject an antiseptic or antibiotic agent.

In an embodiment, the disclosure provides a method of accelerating and/ or promoting wound healing in a subject in need thereof, the method comprising applying magnetic field treatment at the wound, wherein the magnetic field treatment comprises at least two phases, wherein in each phase a different type of periodic wave is applied, wherein the periodic waves differ in their periodic waveform, their frequency and/or their intensity. Preferably, the method comprises the administration or application of an antibiotic or antiseptic agent before, concurrently with or after the application of the magnetic field treatment, administering to the subject an antiseptic or antibiotic agent.

The method thus involves applying a magnetic field treatment program to the infected and contaminated areas, i.e. wounds, which affects the bacterial cell wall and cell membrane structures. Without being bound to a theory, the outer barrier of the bacteria becomes porous, allowing antibacterial agents to diffuse better into the bacterial cells. This results in enhanced efficacy of the antibacterial agents, even against resistant or multi-resistant bacterial strains.

The disclosed method provides a novel therapy method for treating infected and contaminated areas, i.e. wounds by selectively eradicating bacteria optionally in combination with antimicrobial agents, such as antibiotics and antiseptics, using magnetic fields. The technology offers a promising approach to address the growing challenge of antibiotic resistance and improve the overall effectiveness of infected contaminated areas, i.e. wound treatments.

The therapy can be applied in various settings, including hospitals, outpatient clinics, home care and other environments.

The disclosed magnetic field treatment can be further optimized by tailoring the magnetic field parameters for specific bacterial strains, wound types, or patient conditions, surfaces and contaminated areas.

The disclosed magnetic field treatment may be adapted to target other types of microorganisms, such as fungi, viruses, or parasites, by adjusting the magnetic field parameters and therapy programs accordingly.

The disclosed magnetic field treatment method may be further optimized by collecting and analyzing data from multiple treatment sessions and using machine learning algorithms to optimize the therapy program for enhanced efficacy.

The invention may also be extended to other applications beyond wound treatment, such as targeting and eliminating biofilms, which are complex i.e. bacteria that can form on various surfaces, including medical devices and implants. Additionally, the technology may be used in synergy with other treatment modalities, such as light-therapy or bacteriophage therapy, to achieve synergistic effects in combating bacterial and other infections and contaminations.

### In vitro methods

The herein disclosed magnetic field treatment can also be used for disinfection of tissues or non-living objects.

The present invention thus also refers to an *in vitro* method of decreasing microbial load of a tissue, the method comprising applying an antibiotic agent (agent) to the tissue prior to, concurrently with, or after applying a magnetic field treatment to the tissue, wherein the magnetic field treatment comprises at least two phases, wherein in each consecutive phase a different type of periodic wave is applied, wherein the periodic waves differ in their periodic waveform, their frequency and/or their intensity.

In yet another aspect, the invention relates to an *in vitro* method of enhancing microbial cell susceptibility to an antibiotic agent (agent), the method comprising applying a magnetic field treatment to the microbial cell, wherein the magnetic field treatment comprises at least two phases, wherein in each consecutive phase a different type of periodic wave is applied, wherein the periodic waves differ in their periodic waveform, their frequency and/or their intensity.

The waveforms used in the *in vitro* methods comprise square, triangle, sinusoidal, sawtooth, pulsed or arbitrary waveforms, preferably square, triangle, sinusoidal, sawtooth waveforms. "Sinusoidal" is interchangeably used with "sinus";

The periodic waves may have an amplitude range of 0.1-100 mT. The amplitude may thus be 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95 or 100 mT.

A periodic wave may comprise a frequency spectrum of between 0.1 and 15000 Hz. A periodic wave may have a frequency of between 0.1 and 15000 Hz. A periodic wave may thus have a frequency of 0.1, 0.2, 0.3, 0.4, 0.5, 0.6, 0.7, 0.8, 0.9, 1, 5, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5500, 6000, 6500, 7000, 7500, 8000, 8500, 9000, 9500, 10000, 11000, 12000, 13000, 14000, or 15000 Hz. The applied magnetic field may have a frequency in the range of 0.1-50, 50-100, 100-500, 500-1000, 500-2000, 500-3000, 1000-3000, 3000-5000, 5000-10000, 10000-15000.

The frequency of a periodic wave may be constant in one phase and may be increasing or decreasing, and/or applied simultaneously with multiple frequences or ranges of frequencies in another phase of the application. A periodic wave with an increasing frequency means that the frequency is 0 Hz or any other starting frequency at a first point and is increased up to an ending frequency. A phase of the treatment may begin with the starting frequency and end with the increased end frequency, wherein within the phase the frequency is increased. An exemplary periodic wave starts at 0 Hz and the frequency is increasing up to 5000 Hz. A periodic wave with a decreasing frequency means that the frequency is 15000 Hz or any other starting frequency at a first point and is decreased down to an ending frequency, optionally 0 Hz. A phase of the treatment may begin with the starting frequency and end with the decreased end frequency, wherein within the phase the frequency is decreased. An exemplary periodic wave starts at 5000 Hz and the frequency is decreasing down to 0 Hz.

An exemplary magnetic field treatment comprises in a first phase a sinus wave at 50 Hz and in a second phase a sinus wave with a frequency of 5000 Hz. Another exemplary magnetic field treatment comprises in a first phase a sinus wave at 50 Hz and in a second phase a sawtooth wave with a frequency of 50 Hz. Another exemplary magnetic field treatment comprises in a first phase a sinus wave at 50 Hz and in a second phase a sawtooth wave with a frequency of 50 Hz. Another exemplary magnetic field treatment comprises in a first phase a sinus wave at 50 Hz and in a second phase a sinus wave with an increasing frequency of from 10 to 1000 Hz. Another exemplary magnetic field treatment comprises in a first phase a sinus wave at 50 Hz and in a second phase a sinus wave with a decreasing frequency of from 1000 to 10 Hz. Another exemplary magnetic field treatment comprises in a first phase a triangle wave at 15000 Hz and in a second phase a sinus wave with a frequency of 15 Hz. Any combination of the above referenced magnetic field features is possible.

The frequency, intensity and/or waveform may be selected based on the microbes present at or in the tissue or the non-living object.

The magnetic field treatment is applied for 0.5 to 24 hours. The treatment may preferably be applied for 10, 15, 20, 25, 30, 35, 40, 45, 50, 55, 60, 70, 80, 90, 100 minutes, or 1, 2, 3, 4, 5, 6, 7, 8, 9, 10, 11, 12, 13, 14, 15, 16, 17, 18, 19, 20, 21, 22, 23 or 24 hours. Preferably, the magnetic field is applied for 0,5-4 hours. If necessary, the antiseptic and magnetic field treatment may be applied in multiple cycles.

The magnetic field treatment may be applied before, concurrently with, or after the application of the antimicrobial agent. The magnetic field treatment may be applied 10-120, preferably 30-60 minutes after the antimicrobial agent has been applied. The antimicrobial incubation time may be dependent from the route of administration and/or the type of antimicrobial used. Thus, incubation times of antibiotics may differ from antiseptics applied at the tissue or the non-living object. The magnetic field treatment may be applied and completed before the antimicrobial agent is applied. Alternatively, the magnetic field treatment is applied concurrently with the agent, i.e., the agent is applied and immediately thereafter, the magnetic field treatment is applied.

In terms of non-living objects, the methods disclosed herein are to use the magnetic field treatment to produce high levels of disinfection on all types of contaminated surfaces.

### EXAMPLES

### Example 1: Two phase magnetic field treatment

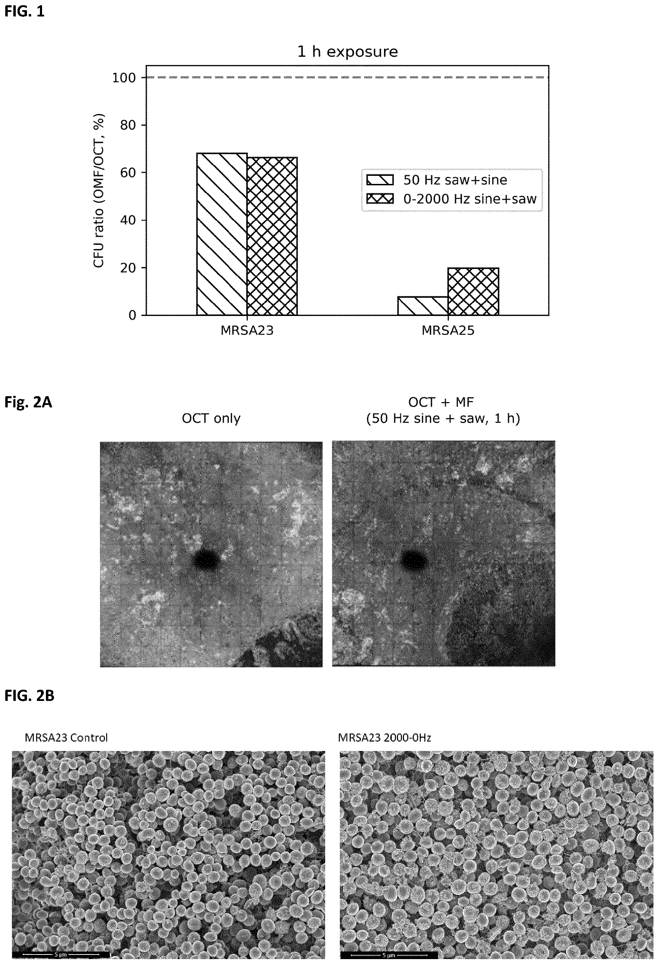

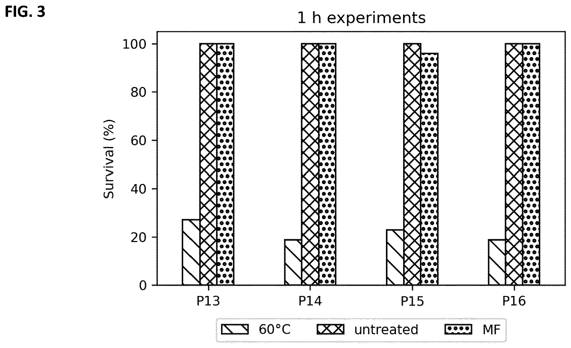

In Example 1, the magnetic field treatment comprises two phases. *Staphylococcus aureus* strains MRSA23 and MRSA25 have been treated with an antiseptic and a two phase alternating magnetic field **(Fig. 3A).** The experiments were performed on cell suspensions in 2 mL Eppendorf tubes. Octenidine (OCT) was chosen in these experiments as the antiseptic. OCT has been used in sub-MIC (concentrations below the Minimal Inhibitory Concentration). Such sub-MIC values for each *Staphylococcus* strain were experimentally selected prior performance of the experiment. Each of the setups consisted of a 1 hour exposure time. For the first experimental setup, the applied magnetic field was an AMF with a sawtooth waveform with a frequency of 50 Hz for 30 min followed by a sinus wave with a frequency of 50 Hz for another 30 min. For the second experimental setup, the applied magnetic field was an AMF with a sine wave with a frequency from 0 Hz to 2000 Hz, i.e. the frequency of the applied magnetic field was constantly increased up to 2000 Hz during an exposure time of 30 min. This treatment was followed by a sawtooth wave with a frequency from 0 Hz to 2000 Hz, i.e. the frequency of the applied magnetic field was again constantly increased from 0 Hz up to 2000 Hz during an exposure time of 30 min. The data presented in the Figure concern the differences in colony forming units (CFU) between *Staphylococcus* suspensions treated with the sub-MIC concentration of OCT combined with the respective MF treatment ("OMF" group) and *Staphylococcus* suspensions exposed to the sub-MIC concentration of OCT only ("OCT" group). The data in **Fig. 1** are average values obtained from nine experimental repeats.

### Example2: Disruption of Biofilm

Example 2 aims for the analysis of the effect of the antiseptic/AMF treatment on a biofilm. Thus, a *S*. *aureus* (strain ATCC6538) biofilm was developed. In the first setting, the biofilm was treated with octenidine (sub-MIC concentration) only. In the second setting, the biofilm was treated with octenidine (sub-MIC concentration) and AMF was applied with a two phase scheme: sinus wave with a frequency of 50 Hz for 30 min directly followed by a sawtooth wave with a frequency of 50 Hz for another 30 min. The biofilm was analyzed for the vitality of the biofilm-forming cells using a spinning disk confocal Microscope (Zeiss Observer SD, magn. 4x) **(****Fig. 2A****).** Each picture consist of 121 field of vision covering about 80% of the whole biofilm. The black dot in the middle of the picture is a marking point on the external surface of the cell culture plate which served as a navigation point for the tiling procedure. It could be observed that the relative number of dead biofilm-forming cells (stained red/orange - here dark grey) was increased whereas the number of living cells (stained green - here light grey) was decreased in the OCT+ MF setting compared to the OCT setting.

The experiment was repeated for analysis of the disruption of the biofilm using a scanning electron microscope (SEM) **(****Fig. 2B****).** For this experiment, the biofilm (MRSA23) was treated with octenidine (sub-MIC concentration) only in the first setting, whereas in the second setting, the biofilm was treated with octenidine (sub-MIC concentration) and AMF was applied with a three phase scheme: sinus wave with a frequency of 50 Hz for 10 min directly followed by a sawtooth wave with a constantly decreasing frequency of 2000-0 Hz for 10 min, directly followed by a sinus wave with a frequency of 50 Hz for another 10 min. The images prove an increased number of MRSA23 cells with deformed shape and decreased turgor due to exposure to the additional AMF compared to the antiseptic-only -treated cells. SEM, magnification 10000x.

### Example 3: Biocompatibility

In Example 3, the biocompatibility of the exposure to alternating magnetic fields was examined. For this, *Galleria mellonella* larvae have been exposed to an alternating magnetic field treatment.

Vitality testing was based on the following experimental groups: untreated larvae, larvae kept at 60°C (negative control), larvae treated with a multiple phase MF treatment for 1 hour. The AMF treatment for each of the experimental groups P13-P16 is explained in **Table 1.** Each experimental group contained 48 larvae.

Imago testing was conducted on the experimental groups from the vitality testing (Fig. 3). After the respective exposure time, the larvae was observed for transformation into imago forms (the last stage an insect attains during its metamorphosis, its process of growth and development - here: moth). The examples show that AMF treatment had no impact on the larva development.

**Table 1: Experimental groups**

| | |
|---|---|
| *P13* | *SIN+SWT*/*0-2000Hz* |
| *P14* | *SIN+SWT*/*50Hz* |
| *P15* | *SWT+SIN*/*50Hz* |
| *P16* | *SWT+SIN*/*0-2000Hz* |

## Claims

1. An antiseptic or antibiotic agent (agent) for use in treating a wound in a subject, wherein the subject is to receive a magnetic field treatment at the wound before, after or concurrently with the application of the agent, and wherein the magnetic field treatment comprises at least two phases of periodic waves, wherein in each consecutive phase a different type of periodic wave is applied, wherein the periodic waves differ in their waveform, their frequency and/or their intensity.

2. The agent for use in treating a wound of claim 1, wherein the magnetic field treatment comprises periodic waves with an amplitude range of 0.1-100 mT.

3. The agent for use in treating a wound according to claims 1 or 2, wherein the magnetic field treatment comprises periodic waves with a frequency spectrum between 0.1 and 15000 Hz and/or with a variation of the frequency.

4. The agent for use in treating a wound of claim 3, wherein the magnetic field treatment comprises in one phase periodic waves with a constant frequency and in a further phase periodic waves with increasing or decreasing frequencies, and/or simultaneous application of multiple frequences or ranges of frequencies.

5. The agent for use in treating a wound of any one of claims 1 to 4, wherein the periodic waveforms comprise sinusoidal, sawtooth, square, triangle, and/or arbitrary waveforms.

6. The agent for use in treating a wound of any one of claims 1 to 5, wherein the magnetic field treatment is applied for 0.5 to 12 hours, preferably for 0.5, 1 or 2 hours.

7. The agent for use in treating a wound of any one of claims 1 to 6, wherein the agent is selected from the group comprising alcohols,, iodine compounds, biguanides, peroxides, silver compounds, and octenidine dihydrochloride, beta-lactams, macrolides, fluoroquinolones, tetracyclines, aminoglycosides, sulfonamides, glycopeptides, oxazolidinones, lincosamides, nitroimidazoles, polymyxins, streptogramins, ansamycins, lipopeptides, and plant-based antimicrobials, or mixtures thereof.

8. The agent for use in treating a wound of any one of claims 1 to 7, wherein the magnetic field treatment is applied to the wound 30-60 min after the application of the agent.

9. An *in vitro* method of decreasing microbial load of a tissue, the method comprising applying an antiseptic or antibiotic agent (agent) to the tissue prior to, concurrently with, or after applying a magnetic field treatment to the tissue, wherein the magnetic field treatment comprises at least two phases, wherein in each consecutive phase a different type of periodic wave is applied, wherein the periodic waves differ in their periodic waveform, their frequency and/or their intensity.

10. An *in vitro* method of enhancing microbial cell susceptibility to an antiseptic or antibiotic agent (agent), the method comprising applying a magnetic field treatment to the microbial cell, wherein the magnetic field treatment comprises at least two phases, wherein in each consecutive phase a different type of periodic wave is applied, wherein the periodic waves differ in their periodic waveform, their frequency and/or their intensity.

11. The *in vitro* method of claim 9 or 10, wherein the periodic waveforms comprise sinusoidal, sawtooth, square, triangle, and arbitrary waveforms.

12. The *in vitro* method of any of claims 9 to 11, wherein the magnetic field treatment comprises periodic waves with an amplitude range of 0.1-100 mT.

13. The *in vitro* method of any of claims 9 to 12, wherein the magnetic field treatment comprises periodic waves with a frequency spectrum of between 0,1 and 15000 Hz and/or with a variation of the frequency.

14. The *in vitro* method of any of claims 9-13, wherein the magnetic field treatment is applied for 0.5 to 24 hours, preferably for 0.5, 1 or 2 hours.

15. The *in vitro* method of any of claims 9-14, wherein the agent is selected from the group comprising alcohols, halogenated phenols, quaternary ammonium compounds, iodine compounds, biguanides, peroxides, silver compounds, octenidine dihydrochloride, and aldehydes, beta-lactams, macrolides, fluoroquinolones, tetracyclines, aminoglycosides, sulfonamides, glycopeptides, oxazolidinones, lincosamides, nitroimidazoles, polymyxins, streptogramins, ansamycins,lipopeptides and plant-based antimicrobials.
